# FASCICULE DE BREVET EUROPEEN

(11) **EP 2 146 713 B1**
(45) Date de publication et mention de la délivrance du brevet: **09.11.2011**
(21) Numéro de dépôt: 08787960.7
(22) Date de dépôt: 16.04.2008
(51) Int. Cl.: A61K 31/444, A61P 25/00

(54) **UTILISATION DU 4-CYCLOPROPYLMETHOXY-N-(3,5-DICHLORO-1-OXYDO-4- PYRIDIN-4-YL)-5-(METHOXY)PYRIDINE-2-CARBOXAMIDE POUR LE TRAITEMENT DES TRAUMATISMES CRANIENS**
VERWENDUNG VON 4-CYCLOPROPYLMETHOXY-N-(3,5-DICHLOR-1-OXIDOPYRIDIN-4-YL)-5-(METHOXY)-PYRIDIN-2-CARBOXAMID ZUR BEHANDLUNG VON SCHÄDELTRAUMATA
USE OF 4-CYCLOPROPYLMETHOXY-N-(3,5-DICHLORO-1-OXIDOPYRIDIN-4-YL)-5-(METHOXY)PYRIDINE-2-CARBOXAMIDE FOR THE TREATMENT OF CRANIAL TRAUMAS

(30) Priorité: 19.04.2007 FR 0702852
(43) Date de publication de la demande: 27.01.2010
(73) Titulaire: SANOFI, 75013 Paris (FR)
(72) Inventeur: DELAY-GOYET, Philippe, F-75013 Paris (FR); PERRON, Corinne, F-75013 Paris (FR)
(74) Mandataire: Gaslonde, Aude
(86) Numéro de dépôt international: PCT/FR2008/000531
(87) Numéro de publication internationale: WO 2008/145838

(56) Documents cités:
- WO-A-95/04045
- WO-A-95/20578
- WO-A-96/31476
- WO-A-02/069905
- WO-A-2004/067006

## Description

La présente invention a pour objet l'utilisation du 4-cyclopropylméthoxy-*N*-(3,5-dichloro-1-oxydo-pyridin-4-yl)-5-(méthoxy)pyridine-2-carboxamide, sous forme d'hydrate, de solvate, de base ou de sel d'addition à un acide, pour la préparation d'un médicament destiné au traitement des traumatismes cérébraux.

Le 4-cyclopropylméthoxy-*N*-(3,5-dichloro-1-oxydo-pyridin-4-yl)-5-(méthoxy)pyridine-2-carboxamide ou encore appelé *N*-(3,5-dichloro-1-oxydo-4-pyridinio)-4-cyclopropylméthoxy-5-méthoxypyridine-2-carboxamide, est connu pour entrer dans la composition de médicaments destinés au traitement de différentes pathologies dont notamment les inflammations articulaires, l'arthrite, l'arthrite rhumatoïde. Ce composé, sous forme hémihydrate, est décrit par exemple dans le document WO95/04045 (composé référencé FR).

Il existe un besoin de trouver des médicaments permettant de traiter les patients souffrant de traumatismes cérébraux. Des études ont montré chez l'animal qu'une voie possible est l'administration de composés inhibant les phosphodiesterases 4 (PDE 4), tels que par exemple le rolipram. Toutefois, des études cliniques ont montré que ce composé, ainsi que d'autres inhibiteurs de PDE 4, induit des effets émétisants qui ne permettent pas son utilisation en thérapie.

On a trouvé maintenant que le 4-cyclopropylméthoxy-*N*-(3,5-dichloro-1-oxydo-pyridin-4-yl)-5-(méthoxy)pyridine-2-carboxamide peut être utilisé dans le traitement des traumatismes cérébraux, tout en évitant les effets émétisants à des doses thérapeutiques acceptables.

Un premier objet de l'invention concerne donc l'utilisation du 4-cyclopropylméthoxy-*N-*(3,5-dichloro-1-oxydo-pyridin-4-yl)-5-(méthoxy)pyridine-2-carboxamide pour la préparation d'un médicament destiné au traitement des traumatismes cérébraux.

Selon un mode d'exécution de l'invention, l'utilisation du 4-cyclopropylméthoxy-*N*-(3,5-dichloro-1-oxydo-pyridin-4-yl)-5-(méthoxy)pyridine-2-carboxamide peut se faire sous forme de base ou de sel d'addition à un acide.

Les sels utilisables dans le cadre de l'invention peuvent être préparés avec des acides pharmaceutiquement acceptables, mais les sels d'autres acides utiles, par exemple, pour la purification ou l'isolement du 4-cyclopropylméthoxy-*N*-(3,5-dichloro-1-oxydo-pyridin-4-yl)-5-(méthoxy)pyridine-2-carboxamide, font également partie de l'invention.

L'utilisation du 4-cyclopropylméthoxy-*N*-(3,5-dichloro-1-oxydo-pyridin-4-yl)-5-(méthoxy)pyridine-2-carboxamide selon l'invention peut se faire également sous forme d'hydrate ou de solvate. Par hydrate ou solvate on entend l'association ou la combinaison de une ou plusieurs molécules de 4-cyclopropylméthoxy-*N*-(3,5-dichloro-1-oxydo-pyridin-4-yl)-5-(méthoxy)pyridine-2-carboxamide avec une ou plusieurs molécules d'eau ou de solvant.

Au sens de la présente invention, on entend par traumatismes cérébraux les traumatismes d'origine externe comme par exemple les traumatismes crâniens, dus notamment à un choc, un accident de la circulation, une chute ou un écrasement.

Un second objet de l'invention concerne une composition pharmaceutique comprenant, à titre de principe actif, le 4-cyclopropylméthoxy-*N*-(3,5-dichloro-1-oxydo-pyridin-4-yl)-5-(méthoxy)pyridine-2-carboxamide, et un ou plusieurs excipients pharmaceutiquement acceptables.

La composition utilisée selon l'invention comprend une dose efficace du principe actif.

Par exemple, les doses journalières de principe actif utilisable selon l'invention sont de 0,001 à 10 mg/jour.

Selon la pratique habituelle, le dosage approprié à chaque patient est déterminé par le médecin selon le mode d'administration, l'âge, le poids et la réponse dudit patient.

Les doses dépendent de l'effet recherché, de la durée du traitement et de la voie d'administration utilisée.

Il peut y avoir des cas particuliers où des dosages plus élevés ou plus faibles sont appropriés. De tels dosages ne sortent pas du cadre de l'invention.

Les excipients sont choisis selon la forme pharmaceutique et le mode d'administration souhaité, parmi les excipients habituels qui sont connus de l'homme du métier.

La composition peut être administrée par voie orale, parentérale ou rectale.

Les formes unitaires d'administration appropriées comprennent les formes par voie orale telles que les comprimés, les gélules molles ou dures, les poudres, les granules et les solutions ou suspensions orales, les formes d'administration sublinguale, buccale, intratrachéale, intraoculaire, intranasale, par inhalation, les formes d'administration topique, transdermique, sous-cutanée, intra musculaire, intraveineuse ou intrathécale, les formes d'administration rectale et les implants. Pour l'application topique, on peut utiliser les principes actifs selon l'invention dans des crèmes, gels, pommades ou lotions.

Lorsqu'on prépare une composition sous forme de comprimé, on mélange le principe actif avec un ou plusieurs excipients pharmaceutiques, tels que la gélatine, l'amidon, le lactose, le stéarate de magnésium, le talc, la silice, la gomme arabique, le mannitol, la cellulose microcristalline, l'hypromellose ou analogues.
On peut enrober les comprimés de saccharose, d'un dérivé cellulosique ou d'autres matières adaptées à l'enrobage. Les comprimés peuvent être réalisés par différentes techniques, telles que la compression directe, la granulation sèche ou humide ou la fusion à chaud.
On peut également obtenir une composition pharmaceutique sous forme de gélule en mélangeant le principe actif avec un diluant et en versant le mélange obtenu dans des gélules molles ou dures.
Pour une administration parentérale, on utilise des suspensions aqueuses, des solutions salines isotoniques ou des solutions stériles et injectables qui contiennent des agents pharmacologiquement compatibles, par exemple le propylèneglycol ou le butylèneglycol.

A titre d'exemple, une forme unitaire d'administration de 4-cyclopropylméthoxy-N-(3,5-dichloro-1-oxydo-pyridin-4-yl)-5-(méthoxy)pyridine-2-carboxamide sous forme de comprimé comprend les ingrédients suivants :

| | |
|---|---|
| 4-cyclopropylméthoxy-*N*-(3,5-dichloro-1-oxydo-pyridin-4-yl)-5-(méthoxy)pyridine-2-carboxamide | 1 mg |
| Mannitol | 224 mg |
| Croscarmellose sodique | 5 mg |
| Amidon de maïs | 15 mg |
| Hydroxypropyl-méthylcellulose | 2 mg |
| Stéarate de magnésium | 3 mg |

Les effets du 4-cyclopropylméthoxy-*N*-(3,5-dichloro-1-oxydo-pyridin-4-yl)-5-(méthoxy)pyridine-2-carboxamide utilisé selon l'invention ont été évalués dans un modèle de traumatisme crânien.

### Expérience 1 : Effet d'un traitement chronique du composé de l'invention dans le test du CFR (« Conditioned Freezing Response ») après un traumatisme crânien pariétal.

Après anesthésie générale, des rats (Sprague-Dawley, mâles, 150-200g, Charles River) sont placés en contention stéréotaxique. Une crâniotomie est effectuée au niveau du cortex pariétal (AP: 3,5 mm; LAT: 7mm et 3,5 mm en dessous du toit crânien). Un cathéter relié à une pompe HPLC est placé au contact de la dure-mère et une pression de 0,073 Kpsi est exercée.

Les animaux sont traités par voie intra-veineuse 4h après la chirurgie (J0) par administration d'une solution de 4-cyclopropylméthoxy-*N*-(3,5-dichloro-1-oxydo-pyridin-4-yl)-5-(méthoxy)pyridine-2-carboxamide dans le véhicule (PEG200 / sérum physiologique (Nacl 0,9%)), à la dose de 0,05 mg/kg.

A partir de J1 et jusqu'à J21 post-chirurgie les animaux sont traités avec une solution contenant du 4-cyclopropylméthoxy-*N*-(3,5-dichloro-1-oxydo-pyridin-4-yl)-5-(méthoxy)pyridine-2-carboxamide dans le véhicule (méthylcellulose (MC) (0,6%) + tween-80 (0,5%) dans l'eau) par voie per os, 2 fois par jour, à la dose totale journalière de 0.1 mg/kg.

Le déficit dans le CFR a été mesuré aux jours J23 et J24 post-chirurgie.

A J23 l'animal est placé dans une boîte où il reçoit un choc électrique de 0,6 mA pendant 1,5 s.

A J24 l'animal est de nouveau placé dans la même boîte et la durée d'immobilité est mesurée pendant 3 minutes.

Une lésion induite par un traumatisme du cortex pariétal réduit significativement le temps d'immobilité.

Le traitement avec le 4-cyclopropylméthoxy-*N*-(3,5-dichloro-1-oxydo-pyridin-4-yl)-5-(méthoxy)pyridine-2-carboxamide à la dose de 0,1 mg/kg/jour inhibe à 100% le déficit induit par un traumatisme crânien (p<0,001 en comparaison avec les animaux traumatisés recevant le véhicule).

### Expérience 2 : Evaluation des effets émétisants du 4-cyclopropylméthoxy-N-(3,5-dichloro-1-oxydo-pyridin-4-yl)-5-(méthoxy)pyridine-2-carboxamide.

Le pouvoir émétique du 4-cyclopropylméthoxy-*N*-(3,5-dichloro-1-oxydo-pyridin-4-yl)-5-(méthoxy)pyiridine-2-carboxamide a été évalué chez le furet. Deux groupes de furets ont été utilisés, le premier recevant le véhicule (PEG200) et le second le 4-cyclopropylméthoxy-*N*-(3,5-dichloro-1-oxydo-pyridin-4-yl)-5-(méthoxy)pyridine-2-carboxamide en solution dans le véhicule (PEG 200), par gavage oral. Les animaux ont été observés continuellement pendant les 2 heures suivant l'administration puis une fois par heure jusqu'à 6 heures après l'administration. Les signes cliniques (en particulier hauts-le-coeur et vomissements) ont été notés.

Administré à 0,1 mg/kg, le 4-cyclopropylméthoxy-N-(3,5-dichloro-1-oxydo-pyridin-4-yl)-5-(méthoxy)pyridine-2-carboxamide n'induit aucun haut-le-coeur ni vomissement chez les 5 furets traités.

Ces résultats montrent que l'administration d'une dose thérapeutique de 4-cyclopropylméthoxy-*N*-(3,5-dichloro-1-oxydo-pyridin-4-yl)-5-(méthoxy)pyridine-2-carboxamide pour traiter des traumatismes cérébraux, n'entraîne pas d'effet émétique.

### EXEMPLE 3 : Evaluation des effets émétisants du (R)-(-)-rolipram (((4R)-4-[3-(Cyclopentyloxy)-4-methoxyphenyl]pyrrolidine-2-one)).

Le pouvoir émétique du (*R*)-(-)-rolipram a été évalué chez le furet. Deux groupes de furets ont été utilisés, le premier recevant le véhicule (PEG200) et le second le (4R)-4-[3-(cyclopentyloxy)-4-methoxyphenyl]pyrrolidine-2-one en solution dans le véhicule (PEG 200), par gavage oral, aux doses de 0,05 mg/kg et de 0,1 mg/kg. Les animaux ont été observés continuellement pendant les 2 heures suivant l'administration puis une fois par heure jusqu'à 6 heures après l'administration. Les signes cliniques ont été notés.

Administré à 0,05 mg/kg et 0,1 mg/kg, le (*R*)-(-)-rolipram induit des vomissements chez les furets traités.

Les résultats de l'exemple 3 montrent que l'administration d'une dose thérapeutique de (*R*)-(-)-Rolipram entraîne des effets émétiques.

Ainsi, le 4-cyclopropylméthoxy-*N*-(3,5-dichloro-1-oxydo-pyridin-4-yl)-5-(méthoxy)pyridine-2-carboxamide est utile dans la préparation d'un médicament pour le traitement des traumatismes cérébraux, comme par exemple les traumatismes survenant lors d'une chute, un choc ou un accident automobile, tout en évitant d'éventuels effets émétisants à des doses thérapeutiques acceptables.

## Revendications

1. Utilisation du 4-cyclopropylméthoxy-*N*-(3,5-dichloro-1-oxydo-pyridin-4-yl)-5-(méthoxy)pyridine-2-carboxamide, sous forme d'hydrate, de solvate, de base ou de sel d'addition à un acide, pour la préparation d'un médicament destiné au traitement des traumatismes cérébraux.

2. Utilisation selon la revendication 1, **caractérisée en ce que** le 4-cyclopropylméthoxy-*N*-(3,5-dichloro-1-oxydo-pyridin-4-yl)-5-(méthoxy)pyridine-2-carboxamide est sous forme de base.

## Claims

1. Use of 4-cyclopropylmethoxy-*N*-(3,5-dichloro-1-oxidopyridin-4-yl)-5-(methoxy)pyridine-2-carboxamide, in the form of a hydrate, of a solvate, of a base or of an addition salt with an acid, for the preparation of a medicament for use in the treatment of cerebral traumas.

2. Use according to Claim 1, **characterized in that** the 4-cyclopropylmethoxy-*N*-(3,5-dichloro-1-oxidopyridin-4-yl)-5-(methoxy)pyridine-2-carboxamide is in the form of a base.

## Patentansprüche

1. Verwendung von 4-Cyclopropylmethoxy-*N*-(3,5-dichlor-1-oxidopyridin-4-yl)-5-(methoxy)pyridin-2-carboxamid in Hydrat-, Solvat-, Basen- oder Säureadditionssalzform zur Herstellung eines Arzneimittels zur Behandlung von Hirntraumata.

2. Verwendung nach Anspruch 1, **dadurch gekennzeichnet, daß** das 4-Cyclopropylmethoxy-*N*-(3,5-dichlor-1-oxidopyridin-4-yl)-5-(methoxy)pyridin-2-carboxamid in Basenform vorliegt.
